Europäisches Patentamt

European Patent Office

Office européen des brevets

(11) Veröffentlichungsnummer: **0 191 284**
**B1**

(12) **EUROPÄISCHE PATENTSCHRIFT**

(45) Veröffentlichungstag der Patentschrift: **17.10.90**

(21) Anmeldenummer: **86100121.2**

(22) Anmeldetag: **07.01.86**

(51) Int. Cl.⁵: **G 01 N 33/573,**
G 01 N 33/577, C 07 K 15/00,
C 12 N 5/00, C 12 N 15/00,
C 12 P 21/00, C 12 Q 1/40,
C 12 Q 1/28 // (C12P21/00,
C12R1:91)

(54) Spezifisch hemmender Speichel-alpha-Amylase monoklonaler Antikörper und dessen Verwendung zur Bestimmung von Pankreas-alpha-Amylase.

(30) Priorität: **09.01.85 DE 3500526**

(43) Veröffentlichungstag der Anmeldung:
**20.08.86 Patentblatt 86/34**

(45) Bekanntmachung des Hinweises auf die
Patenterteilung:
**17.10.90 Patentblatt 90/42**

(84) Benannte Vertragsstaaten:
**AT BE CH DE FR GB IT LI LU NL SE**

(56) Entgegenhaltungen:
**EP-A-0 150 309**
**EP-A-0 152 305**

**PATENTS ABSTRACTS OF JAPAN, Band 8, Nr.
15 (C-206) 1452r, 21. Januar 1984; & JP - A - 58
183 098**

Die Akte enthält technische Angaben, die nach
dem Eingang der Anmeldung eingereicht
wurden und die nicht in dieser Patentschrift
enthalten sind.

(73) Patentinhaber: **BOEHRINGER MANNHEIM
GMBH
Patentabteilung, Abt. E Sandhofer Strasse 112-
132 Postfach 31 01 20
D-6800 Mannheim 31 Waldhof (DE)**

(72) Erfinder: **Naujoks, Kurt, Dr. rer. nat.
Hiltlstrasse 15
D-8035 Gauting (DE)**
Erfinder: **Wulff, Karl, Dr. rer. nat.
Pütrichstrasse 18
D-8120 Weilheim (DE)**
Erfinder: **Gerber, Martin, Dr. rer. nat.
Ignaz-Manz-Strasse 1
D-8120 Weilheim-Unterhausen (DE)**

(74) Vertreter: **Weickmann, Heinrich, Dipl.-Ing. et al
Patentanwälte Dipl.-Ing. H.Weickmann Dipl.-
Phys.Dr. K.Fincke Dipl.-Ing. F.A.Weickmann
Dipl.-Chem. B. Huber Dr.-Ing. H. Liska Dipl.-
Phys.Dr. J. Prechtel Postfach 860820
D-8000 München 86 (DE)**

**Beschreibung**

Die Erfindung betrifft ein Verfahren zur spezifischen Bestimmung von Pankreas-alpha-Amylase neben Speichel-alpha-Amylase.

Alpha-Amylase (E.C.3.2.1.1) baut 1,4-d-glucosidisch verknüpfte Oligo- und Polysaccharid überwiegend durch zufällige Hydrolyse der 1,4-alpha-glucosidischen Bindungen zu Maltose und Malto-oligosacchariden ab. Das Enzym hat neben der industriellen Fermentationstechnik erhebliche Bedeutung im Rahmen der klinischen Analytik und Diagnostik. Bei zahlreichen Erkrankungen verändert sich nämlich der alpha-Amylasegehalt in den Körperflüssigkeiten wie Serum, Harn oder Duodenalsekret beträchtlich. Im Körper kommen jedoch im wesentlichen zwei alpha-Amylaseenzyme vor, das Pankreasenzym und das Speichelenzym. Da diagnostische Bedeutung nur dem Pankreasenzym zukommt, stellt sich die Aufgabe, diese beiden (neben selten und nur in geringer Menge auftretenden weiteren Isoenzymen) alpha-Amylasen analytisch zu differenzieren. Die Schwierigkeit besteht hierbei darin, daß die beiden multiplen Formen ähnlichen Aufbau besitzen und immunologisch identisch sind (K. Lorentz, Laboratoriumsblätter 32: 118 (1982)). Zur Eliminierung der Aktivität des Speichelenzyms ist es bekannt, Adsorbtion an Anionenaustauscher, Hemmung durch ein Weizenprotein oder Elektrophorese bzw. Elektrofokusierung anzuwenden. Diese Verfahren sind jedoch entweder in ihrer Trennwirkung unbefriedigend oder für eine Routinediagnostik zu aufwendig. Unter den genannten Methoden ist allein das in Clin. Chem. 28/7, 1525-1527 (1982) beschriebene Verfahren der Hemmung des Enzyms vom Speicheltyp durch einen aus Weizenkeimen gewonnenen Inhibitor mit einem für die Routinediagnose akzeptablen Zeitaufwand verbunden, jedoch ist die Selektivität unbefriedigend. Auch bei der optimalen Inhibitorkonzentration bleiben etwa 13% der Aktivität des Speicheltyp-Enzyms erhalten, während die Aktivität des Pankreasenzyms auf etwa 81% verringert wird.

In der älteren, nicht vorveröffentlichten Europäischen Patentanmeldung Nr. EP-A-150 309 wird bereits vorgeschlagen, die Pankreas-alpha-Amylase neben der Speichelalpha-Amylase zu bestimmen, indem man in Gegenwart eines monoklonalen Antikörpers arbeitet, der mit Speichel-alpha-Amylase reagiert und hierbei eine Kreuzreaktivität von 5% oder weniger gegenüber Pankreas-alpha-Amylase aufweist. Mit diesem monoklonalen Antikörper ist es bei Zusatz eines präzipitierenden Agens auch möglich, einen unlöslichen Komplex mit der Speichel-alpha-Amylase zu bilden, den man aus der Lösung abtrennen kann, so daß nur das Pankreasenzym in der Lösung zurückbleibt und dort bestimmt werden kann. Alternativ ist es möglich, den monoklonalen Antikörper in immobilisierter Form zu verwenden und auf diese Weise die Speichel-Amylase abzutrennen. In beiden Fällen ist es jedoch erforderlich, eine unlösliche Phase zu bilden und von der löslichen Phase zu trennen.

Der Erfindung liegt daher die Aufgabe zugrunde, diesen Nachteil zu beseitigen und ein Verfahren und ein Reagenz zu schaffen, welches eine rasche, einfache und zuverlässige Bestimmung der Pankreas-aipha-Amylase neben der alpha-Amylase vom Speicheltyp in Körperflüssigkeiten ermöglicht, ohne hierbei eine Phasentrennung vornehmen zu müssen.

Gelöst wird diese Aufgabe erfindungsgemäß durch ein Verfahren zur spezifischen Bestimmung von Pankreas-alpha-Amylase neben Speichel-alpha-Amylase in Körperflüssigkeiten, insbesondere in Serum, Plasma, Duodenalsaft oder Urin, durch Umsetzung mit einem System zum Nachweis von alpha-Amylase in Gegenwart eines monoklonalen Antikörpers, der mit Speichel-alpha-Amylase reagiert, welches dadurch gekennzeichnet ist, daß man einen monoklonalen Antikörper verwendet, welcher das Speichelenzym spezifisch hemmt, das Pankreasenzym aber nicht mehr als 50% inhibiert.

Das erfindungsgemäße Verfahren beruht auf der sehr überraschenden Auffindung eines monoklonalen Antikörpers, welcher das Speichelenzym hemmt, nicht jedoch das Pankreasenzym. Dies war nicht zu erwarten, da es bekannt war, daß das Speichel- und das Pankreasenzym immunologisch identisch sind (Gerhard Pfleiderer, Lab. Med. 7, 189-193; K. Lorentz loc. cit.). So gibt Morton K. Schwarz in "Immunoassay of Enzymes — an Overview" (1983), Seite 4 bis 9, z. B. an, daß Antikörper gegen humane Speichel-alpha-Amylase das Enzym vom Speicheltyp zu 78%, das Pankreasenzym zu 75% hemmen. Es war daher nicht vorhersehbar, daß es möglich sein würde, einen monoklonalen Antikörper zu entwickeln, welcher eine praktisch quantitative Unterscheidung der beiden Enzyme durch selektive hochspezifische Hemmung ermöglichen würde. Dabei wird eine Hemmung von 95% und mehr für das Speichelenzym bei den bevorzugten Antikörpern erzielt.

Der neue, für das Verfahren der Erfindung verwendete, monoklonale Antikörper wurde bei NCACC unter den Nummern (99D12) 84122003 und (89E2) 84122004 hinterlegt (National Collection of Animal Cell Culture, Porton Down, GB).

Er läßt sich erfindungsgemäß erhalten durch Immunisierung von Versuchstieren mit nativer oder modifizierter Speichel-alpha-Amylase, Fusion von B-Lymphocyten der so erhaltenen immunisierten Tiere mit transformierenden Agenzien, Klonierung und Kultivierung der so gebildeten Hybridzellen, welche den monoklonalen Antikörper produzieren und Isolierung des letzteren. Besonders geeignete Tiere für die Herstellung der Speichel-alpha-Amylase-Antikörper sind Ratten und Mäuse. Die Immunisierung erfolgt entweder mit nativer humaner Speichel-alpha-Amylase oder mit modifizierter Speichel-Amylase. Verwendet man natives Enzym, so eignen sich hierzu die handelsüblichen, elektrophoretisch homogenen Präparate. Chemisch modifizierte Speichel-alpha-Amylase läßt sich ebenfalls nach bekannten Methoden der Enzymmodifikation erhalten, wie sie z. B. in der DE-AS 25 43 994 näher beschrieben sind. Geeignete

# EP 0 191 284 B1

Modifizierungsmittel sind beispielsweise N-Bromsuccinimid (NBS) unter Oxidation von Tryptophangruppen am Protein (BBA 143 (1967) 462-472), Carboxymethylierung mit Jodacetat (IAA), die hauptsächlich am Histidin angreift bzw. Nitrierung mit Tetranitromethan (TNM) (J. Biol. Chem. 238 (1963) 3307) sowie Diazotierung mit diazotierter Sulfanilsäure (Meth. Enzymol. 25 (1972) 515-531). Am besten geeignet erwies sich dabei das mit RNM modifizierte Enzym bei Verwendung von Balb/c-Mäusen oder das native Enzym bei Verwendung von AJ-Mäusen.

Die Immunisierung erfolgt durch übliche Verabreichung des nativen oder modifizierten Enzyms, vorzugsweise in Kombination mit Adjuvanz. Bevorzugt wird als Adjuvanz Aluminiumhydroxid zusammen mit Bordatella pertussis.

Wird für die Immunisierung native Speichel-alpha-Amylase in AJ-Mäusen oder TNM-modifizierte Speichel-alpha-Amylase in Balb/c-Mäusen verwendet, so erfolgt die Immunisierung vorzugsweise über mindestens 9 Monate mit mindestens 7 Immunisierungen (Injektionen I.P.).

Nach erfolgter Immunisierung werden die B-Lymphocyten der immunisierten Tiere nach üblichen Methoden mit transformierenden Agenzien fusioniert. Beispiele für transformierende Agenzien, die im Rahmen der Erfindung verwendet werden können, sind Myelomazellen, transformierende Viren wie z. B. Epstein-Barr-Virus oder die in der deutschen Offenlegungsschrift 32 45 665 beschriebenen Agenzien. Die Fusionierung erfolgt nach dem bekannten Verfahren von Koehler und Milstein (Nature 256 (1975) 495-997). Die hierbei gebildeten Hybridzellen werden in üblicher Weise kloniert, z. B. unter Verwendung eines handelsüblichen Zellsorters, und die erhaltenen Klone, welche den gewünschten monoklonalen Antikörper bilden, gezüchtet. Aufgrund des krebsartigen Wachsens der Hybridzellen lassen sich diese auf unbestimmte Zeit weiterkultivieren und produzieren den gewünschten monoklonalen Antikörper in beliebiger Menge. Mit den so erhaltenen monoklonalen Antikörpern kann die Speichel-alpha-Amylase aus Körperflüssigkeiten quantitativ gehemmt werden, so daß die verbleibende Amylaseaktivität der Pankreas-alpha-Amylase zuzuordnen ist.

Für das erfindungsgemäße Bestimmungsverfahren können die monoklonalen Antikörper als solche oder ihre entsprechende immunologische Eigenschaften aufweisenden Fragmente ($F_c$-Fragmente) verwendet werden. Unter dem Begriff "monoklonaler Antikörper" werden hier daher auch die Fragmente verstanden. Sowohl der komplette Antikörper, als auch seine Fragmente, können in immobilisierter Form verwendet werden.

Der erfindungsgemäß verwendete monoklonale Antikörper weist überraschenderweise gegenüber der Pankreas-alpha-Amylase eine Hemmung von 5% oder weniger auf, erreicht in vielen Fällen eine Hemmung von nur noch 1% und in einigen Fällen sogar eine unterhalb der Meßgrenze liegende Hemmung. Er eignet sich daher anstelle des bisher bekannten, aus Weizenkeimen gewonnenen Hemmstoffes oder des bindenden monoklonalen Antikörpers der oben erwähnten älteren Anmeldung zur spezifischen Bestimmung der Pankreas-alpha-Amylase in Körperflüssigkeiten.

Die Bestimmung der alpha-Amylase als solche erfolgt nach den hierfür bekannten Methoden. Da der erfindungsgemäße monoklonale Antikörper selektiv die alpha-Amylase vom Speicheltyp hemmt und sie so der Enzymaktivitätsbestimmung entzieht, entsprechen die bei der alpha-Amylase-Bestimmung in Gegenwart des monoklonalen Antikörpers erhaltenen Werte der dem Pankreasenzym alleine zukommenden Aktivität.

Bevorzugt wird das erfindungsgemäße Verfahren mit einem System zum Nachweis von alpha-Amylase durchgeführt, welches eine Maltopolyose mit 4 bis 7 Glucoseresten im Molekül, Maltosephosphorylase, ß-Phosphoglucomutase, Glucose-6-phosphatdehydrogenase und NAD enthält.

Ein weiteres, im Rahmen der Erfindung bevorzugt verwendetes System zum Nachweis der alpha-Amylase besteht aus Nitrophenylmaltopolyose mit 4 bis 7 Glucoseresten im Molekül und alpha-Glucosidase.

Ein weiteres bevorzugtes Nachweissystem für alpha-Amylase besteht aus mit bestimmbaren Gruppen modifizierter Stärke. Der Begriff modifizierte Stärke umfaßt beispielsweise Stärke, die mit bestimmbaren Gruppen modifiziert ist, z. B. das als "Phadebas" handelsübliche Produkt der Firma Pharmazia, Schweden sowie das in der DE-OS 28 12 154 beschriebene Produkt sowie im Abbauverhalten veränderte Stärke, beispielsweise Carboxymethylstärke und Grenzdextrine. Alle diese Systeme sind bekannt und bedürfen daher hier keiner näheren Beschreibung.

Für die Durchführung des erfindungsgemäßen Verfahrens wird die Probeflüssigkeit mit dem erfindungsgemäßen Antikörper zweckmäßig inkubiert und danach direkt im üblichen Amylasetest eingesetzt. Die Dauer der Inkubationszeit ist von der Aktivität des eingesetzten Antikörpers abhängig und beträgt vorzugsweise 15 bis 30 Minuten.

Sofern eine Abtrennung der Speichel-alpha-Amylase wünschenswert erscheint, kann der monoklonale Antikörper, sowohl in kompletter Form als auch in Form der Fragmente, auch auf einem festen Träger fixiert vorliegen, beispielsweise auf immunosorptivem Papier oder der Oberfläche von Kunststoffröhrchen bzw. -schläuchen. Auf diese Weise wird die alpha-Amylase vom Speicheltyp an den Träger, also die feste Phase, gebunden.

Es ist auch möglich, ein monoklonales Antikörperpräparat zu verwenden, welches aus den hemmenden monoklonalen Antikörpern der Erfindung, die durch mehrere verschiedene Klone produziert werden, gemischt ist.

Ein weiterer Gegenstand der Erfindung ist ein Reagenz zur spezifischen Bestimmung von Pankreas-

3

alpha-Amylase neben Speichel-alpha-Amylase in Körperflüssigkeiten, insbesondere in Serum, Duodenalsaft, plasma oder Urin, enthaltend ein System zum Nachweis von alpha-Amylase und einen monoklonalen Antikörper gegen Speichel-alpha-Amylase, welches dadurch gekennzeichnet ist, daß es einen monoklonalen Antikörper enthält, welcher das Speichelenzym spezifisch hemmt, das Pankreasenzym aber nicht mehr als 50% inhibiert (Kreuzreaktivität ≲ 50%).

Hinsichtlich des im erfindungsgemäßen Reagenz enthaltenen Systems zum Nachweis von alpha-Amylase und der übrigen Bedingungen gelten die obigen Ausführungen bezüglich des Verfahrens entsprechend. .

Die Erfindung ermöglicht eine einfache und rasche Bestimmung der Pankreas-alpha-Amylase neben alpha-Amylase vom Speicheltyp in Körperflüssigkeiten mit hoher Spezifität und verbessert damit die Möglichkeiten der klinischen Diagnostik.

Die folgenden Beispiele erläutern die Erfindung weiter.

## Beispiel 1

A) AJ-Jäuse werden mit 100 µg humaner Speichelamylase in Aluminiumhydroxid mit BORDETELLA PERTUSSIS immunisiert. In ca. achtwöchigem Rhytmus wird mit je 50 µg humaner Speichelamylase im gleichen Adjuvant drei- bis viermal weiterimmunisiert. Vier Tage vor Fusion wird die letzte Immunisierung mit 50 µg Speichelamylase in physiologischer Kochsalzlösung intravenös durchgeführt.

B) Balb/c-Mäuse werden mit 100 µg humaner TNM-modifizierter Speichelamylase in Aluminiumhydroxid mit BORDETELLA PERTUSSIS immunisiert. In ca. achtwöchigem Rhytmus wird mit je 50 µg humaner TNM-Speichel-amylase im gleichen Adjuvant drei- bis viermal weiterimmunisiert. Vier Tage vor Fusion wird die letzte Immunisierung mit 50 µg Speichelamylase in physiologischer Kochsalzlösung intravenös durchgeführt.

C) Die Fusion der Milzzellen mit Ag8.653 (ATCC CRL 1580) oder SP2/0 (ATCC CRL 1581) Myelomzellen wird nach dem Standardverfahren gemäß J. of Imm. Meth. Vol. 39, 285-308 durchgeführt. Das Fusionsverhältnis Milz zu Myelomzellen ist 5:1. Die Fusionsprodukte werden auf 20 24er-Kulturschalen (von Costar) angesät und mit $5 \times 10^4$ peritoneal-exsudat-Zellen pro Kulturnapf gefüttert. Positive Primärkulturen (siehe Beispiel 3) werden 3 bis 4 Wochen nach Fusion mit Hilfe eines Fluoreszenzaktivierten Zellsorters kloniert. Die Zellen werden einzeln in 96er Costar-Platten abgelegt und mit $2 \times 10^4$ peritoneal-exsudat-Zellen gefüttert. Als Kulturmedium wird verwendet handelsübliches RPMI-1640-Medium mit 10% fötalem Kälberserum (beschrieben in J. A. M. A. 199 (1957) 519.

## Beispiel 2

Modifizierung der alpha-Amylase mit Tetranitromethan (TNM)

5,2 mg Human-Speichelamylase (Sigma) wurden in 1,6 ml Tris-Puffer (50 mM Tris-HC1, 1 mM CaC1$_2$, pH = 8,0) gelöst.

Unter Rühren wurde dazugegeben 10 µl einer 10% (V/V) Lösung von Tetranitromethan (Roth) in abs. Ethanol.

Das Gemisch wurde 12 Stunden bei Raumtemperatur belassen. Dann wurde das Protein durch Dialyse über Nacht gegen den obengenannten Puffer vom überschüssigen Tetranitromethan befreit.

Eine Extinktionsmessung bei 381 nm ergab 2,6 Nitrogruppen je Molekül alpha-Amylase.

## Beispiel 3

Um die Konzentration und Spezifität amylasehemmender Antikörper im Serum immunisierter Mäuse oder im Kulturüberstand der Hybridzellen oder in Ascites zu erfassen, wird ein Amylase-Inhibitions-Test als screening-assay verwendet.

Dazu werden in 96er Elisa-Platten (Firma Nunc) 50 µl Pankreas- oder Speichel-Amylase (etwa 400 mU/ml) in puffer (50 mM Tris, 1% Rinderserumalbumin, 0,1 M NaC1, pH 7,5) vorgelegt und mit 50 µl der zu testenden Lösung (z. B. Kulturüberstand) 20 Minuten vorinkubiert (Raumtemperatur).

Danach wird mit 50 µl Amylasesubstrat (4-Nitrophenyl-maltoheptaosid, Boehringer Mannheim, Best.-Nr. 56 85 89) die Enzymreaktion gestartet. Nach ca. 20 bis 60 Minuten bei Raumtemperatur werden die Extinktionen bei 405 nm in einem Photometer (Elisa-Reader, Kontron, Schweiz) bestimmt.

Folgende Kontrollen werden mitbestimmt:

1. Frisches Kulturmedium (vgl. Beispiel 1 C)
2. Kulturüberstand des Klons der EU-84 114 172.4

4. Weizenkeim-Inhibitor (EP 00 79 793), Konzentrationen 2 µg/ml und 0,2 µg/ml.
In dem oben beschriebenen Testsystem werden folgende Ergebnisse erhalten:

Hemmung %

| | Klon 79 | Weizenkeim-Inhibitor 2 mg/ml | 0,2 µg/ml | 99D12 | 99C11 | 88E8 | 89E2 |
|---|---|---|---|---|---|---|---|
| Speiche-Amylase | 0 | 75 | 40 | 58 | 65 | 67 | 65 |
| Pankreas-Amylase | 0 | 17 | 6,2 | 1,8 | 4,6 | 0,5 | 0 |

Aus ca. 4% aller Primärkulturen werden spezifisch Speichelamylase hemmende Aktivitäten gefunden, deren Kreuzreaktivität kleiner als 10% war.

Beispiel 4

Produktion von Ascites und Bestimmung dessen Aktivität

1 - 2 × 10⁶ Hybridzellen (hergestellt nach Beispiel 1) wurden intraperitoneal in 1 bis 2 ml mit je 0,5 ml Pristan (Sigma, Nr. T 7640) vorbehandelte Mäuse gespritzt. Nach ca. 15 bis 20 Tagen werden pro Maus 3 bis 5 ml Ascites gezapft, der etwa 10 mg IgG/ml enthält.

In einer Konzentration von 200 µg/ml inhibiert der monoklonale Antikörper 89 E2, die humane Speichel-alpha-Amylase (800 mU/ml) zu über 95%, die humane Pankreas-alpha-Amylase zu weniger als 2% (Verfahren analog Beispiel 3).

**Patentansprüche**

1. Verfahren zur spezifischen Bestimmung von Pankreas-alpha-Amylase neben Speichel-alpha-Amylase in Körperflüssigkeiten, insbesondere in Serum, Plasma, Duodenalsaft oder Urin, durch Umsetzung mit einem System zum Nachweis von alpha-Amylase in Gegenwart eines monoklonalen Antikörpers, der mit Speichel-alpha-Amylase reagiert, dadurch gekennzeichnet, daß man einen monoklonalen Antikörper verwendet, welcher das Speichelenzym spezifisch hemmt, das Pankreasenzym aber nicht mehr als 50% inhibiert.

2. Verfahren nach Anspruch 1, dadurch gekennzeichnet, daß man einen monoklonalen Antikörper verwendet, der durch Immunisierung von AJ-Mäusen mit nativer oder modifizierter Speichel-alpha-Amylase, Fusion von B-Lymphocyten der immunisierten Tiere mit transformierenden Agentien, Klonierung und Kultivierung der so gebildeten Hybridzellen und Isolierung der monoklonalen Antikörper aus letzteren erhalten wird.

3. Verfahren nach Anspruch 1, dadurch gekennzeichnet, daß man einen monoklonalen Antikörper verwendet, der durch Immunisierung von Balb/C-Mäusen mit durch Tetranitromethan oder N-Bromsuccinimid, Jodacetat oder diazotierter Sulfanilsäure modifizierter Speichel-alpha-Amylase, Fusion von B-Lymphocyten der immunisierten Tiere mit transformierenden Agentien, Klonierung und Kultivierung der so gebildeten Hybridzellen und Isolierung der monoklonalen Antikörper aus letzteren erhalten wird.

4. Verfahren nach Anspruch 1, dadurch gekennzeichnet, daß man als monoklonalen Antikörper NCACC Nr. 84122003 und NCACC 84122004 verwendet.

5. Verfahren nach Anspruch 2 oder 3, dadurch gekennzeichnet, daß die Immunisierung mit Aluminiumhydroxid und Bordatella pertussis als Adjuvanz durchgeführt wird.

6. Verfahren nach einem der Ansprüche 2 bis 5, dadurch gekennzeichnet, daß als transformierende Agentien Myelomazellen eingesetzt werden.

7. Verfahren nach einem der Ansprüche 1 bis 6, dadurch gekennzeichnet, daß man den monoklonalen Antikörper in immobilisierter Form einsetzt.

8. Verfahren nach einem der vorhergehenden Ansprüche, dadurch gekennzeichnet, daß als System zum Nachweis von Pankreas-alpha-Amylase eine Maltopolyose mit 4 bis 7 Glucoseresten, Maltosephosphorylase, ß-Phosphoglucomutase, Glucose-6-phosphatdehydrogenase und NAD verwendet wird.

9. Verfahren nach einem der Ansprüche 1 bis 7, dadurch gekennzeichnet, daß als System zum Nachweis von Pankreas-alpha-Amylase Nitrophenylmaltopolyose mit 4 bis 7 Glucoseeinheiten im Molekül zusammen mit alpha-Glucosidase verwendet wird.

10. Verfahren nach einem der Ansprüche 1 bis 7, dadurch gekennzeichnet, daß als System zum Nachweis von alpha-Amylase Stärke, die mit bestimmbaren Gruppen modifiziert ist, verwendet wird.

11. Reagenz zur spezifischen Bestimmung von Pankreasalpha-Amylase neben Speichel-alpha-Amylase in Körperflüssigkeiten, insbesondere in Serum, Duodenalsaft Plasma oder Urin, enthaltend ein System zum Nachweis von alpha-Amylase und einen monoklonalen Antikörper gegen Speichel-alpha-Amylase, dadurch gekennzeichnet, daß es einen monoklonalen Antikörper enthält, welcher das Speichelenzym spezifisch hemmt, das Pankreasenzym aber nicht mehr als 50% inhibiert.

12. Reagenz nach Anspruch 11, dadurch gekennzeichnet, daß es als System zum Nachweis von Pankreas-alphy-Amylase eine Maltopolyose mit 4 bis 7 Glucoseresten, Maltosephosphorylase, ß-Phosphoglucomutase, Glucose-6-phosphatdehydrogenase und NAD enthält.

13. Reagenz nach Anspruch 11, dadurch gekennzeichnet, daß es als System zum Nachweis von Pankreas-alpha-Amylase eine Nitrophenylmaltopolyose mit 4 bis 7 Glucoseeinheiten und alpha-Glucosidase enthält.

14. Reagenz nach Anspruch 11, dadurch gekennzeichnet, daß es als System zum Nachweis von Pankreas-alpha-Amylase eine mit bestimmbaren Gruppen modifizierte Stärke enthält.

## Revendications

1. Procédé pour la détermination spécifique de l'alpha-amylase pancréatique en présence d'alpha-amylase de salive dans les liquides biologiques, en particulier dans le sérum, le plasma, le suc duodénal ou l'urine, par réaction avec un système pour la mise en évidence de l'alpha-amylase en présence d'un anticorps monoclonal qui réagit avec l'alpha-amylase de salive, caractérisé en ce qu'on utilise un anticorps monoclonal, lequel inhibe spécifiquement l'enzyme salivaire, mais n'inhibe pas à plus de 50% l'enzyme pancréatique.

2. Procédé selon la revendication 1, caractérisé en ce qu'on utilise un anticorps monoclonal qui est obtenu par immunisation de souris AJ au moyen d'alpha-amylase de salive native ou modifiée, fusion des lymphocytes B des animaux immunisés par des agents transformants, clonage et culture des cellules hybrides ainsi formées et isolement des anticorps monoclonaux à partir de ces dernières.

3. Procédé selon la revendication 1, caractérisé en ce qu'on utilise un anticorps monoclonal qui est obtenu par immunisation de souris Balb/C au moyen d'alpha-amylase de salive modifiée par le tétranitro-méthane ou le N-bromosuccinimide, l'acétate d'iode ou l'acide sulfanilique diazoté, fusion des lymphocytes B des animaux immunisés au moyen d'agents transformants, clonage et culture des cellules hybrides ainsi formées et isolement des anticorps monoclonaux à partir de ces dernières.

4. Procédé selon la revendication 1, caractérisé en ce qu'on utilise comme anticorps monoclonaux les anticorps NCACC no. 84122003 et NCACC 84122004.

5. Procédé selon la revendication 2 ou 3, caractérisé en ce que l'immunisation est effectuée avec de l'hydroxyde d'aluminium et Bordatella pertussis comme adjuvant.

6. Procédé selon l'une des revendications 2 à 5, caractérisé en ce que l'on met en oeuvre, comme agents transformants, des cellules de myélome.

7. Procédé selon l'une des revendications 1 à 6, caractérisé en ce que l'on met l'anticorps monoclonal en oeuvre sous forme immobilisée.

8. Procédé selon l'une des revendications précédentes, caractérisé en ce que l'on utilise comme système pour la mise en évidence de l'alpha-amylase pancréatique un maltopolyose présentant 4 à 7 radicaux glucose, de la maltose phosphorylase, de la β-phosphoglucomutase, de la glucose-6-phosphate deshydrogénase et du NAD.

9. Procédé selon l'une des revendications 1 à 7, caractérisé en ce que l'on utilise comme système pour la mise en évidence de l'alpha-amylase pancréatique du nitrophénylmaltopolyose présentant 4 à 7 unités glucose dans la molécule, ensemble avec de l'alpha-glucosidase.

10. Procédé selon l'une des revendications 1 à 7, caractérisé en ce que l'on utilise comme système pour la mise en évidence de l'alpha-amylase, de l'amidon qui est modifié par des groupes déterminables.

11. Réactif pour la détermination spécifique de l'alpha-amylase pancréatique en présence d'alpha-amylase de salive dans les liquides biologiques, en particulier dans le sérum, le suc duodénal, le plasma ou l'urine, contenant un système pour la mise en évidence de l'alpha-amylase et un anticorps monoclonal contre l'alpha-amylase de salive, caractérisé en ce qu'il contient un anticorps monoclonal, lequel inhibe spécifiquement l'enzyme salivaire, mais n'inhibe pas à plus de 50% l'enzyme pancréatique.

12. Réactif selon la revendication 11, caractérisé en ce qu'il contient, en tant que système pour la mise en évidence de l'alpha-amylase pancréatique, un maltopolyose présentant 4 à 7 radicaux glucose, de la maltose phosphorylase, de la β-phosphoglucomutase, de la glucose-6-phosphate deshydrogénase et du NAD.

13. Réactif selon la revendication 11, caractérisé en ce qu'il contient, comme système pour la mise en évidence de l'alpha-amylase pancréatique, un nitrophénylmaltopolyose présentant 4 à 7 unités glucose et de l'alpha-glucosidase.

14. Réactif selon la revendication 11, caractérisé en ce qu'il contient, en tant que système pour la mise en évidence de l'alpha-amylase pancréatique, un amidon modifié par des groupes déterminables.

**Claims**

1. Process for the specific determination of pancreatic alpha-amylase in the presence of salivary alpha-amylase in body fluids, especially in serum, plasma, duodenal juice or urine, by reaction with a system for the detection of alpha-amylase in the presence of a monoclonal antibody which reacts with salivary alpha-amylase, characterised in that one uses a monoclonal antibody which specifically inhibits the salivary enzyme but inhibits the pancreatic enzyme by not more than 50%.

2. Process according to claim 1, characterised in that one uses a monoclonal antibody which is obtained by immunisation of AJ mice with native or modified salivary alpha-amylase, fusion of B-lymphocytes of the immunised animal with transforming agents, cloning and culturing of the hybrid cells so formed and isolation of the monoclonal antibodies from the latter.

3. Process according to claim 1, characterised in that one uses a monoclonal antibody which is obtained by immunisation of Balb/c mice with salivary alpha-amylase modified by tetranitromethane or N-bromosuccinimide, iodoacetate or diazotised sulphanilic acid, fusion of B-lymphocytes from the immunised animals with transforming agents, cloning and culturing of the hybrid cells so formed and isolation of the monoclonal antibodies from the latter.

4. Process according to claim 1, characterised in that one uses NCACC No. 84122003 or NCACC No. 84122004 as monoclonal antibody.

5. Process according to claim 2 or 3, characterised in that the immunisation is carried out with aluminium hydroxide and Bordatella pertussis as adjuvant.

6. Process according to one of claims 2 to 5, characterised in that myeloma cells are used as transforming agents.

7. Process according to one of claims 1 to 6, characterised in that one uses the monoclonal antibodies in immobilised form.

8. Process according to one of the preceding claims, characterised in that, as system for the detection of pancreatic alpha-amylase, there is used a maltopolyose with 4 to 7 glucose residues, maltose phosphorylase, β-phosphoglucomutase, glucose-6-phosphate dehydrogenase and NAD.

9. Process according to one of claims 1 to 7, characterised in that, as system used for the detection of pancreatic alpha-amylase, there is used nitrophenylmaltopolyose with 4 to 7 glucose residues in the molecule, together with alpha-glucosidase.

10. Process according to one of claims 1 to 7, characterised in that, as system used for the detection of alpha-amylase, there is used starch which is modified with determinable groups.

11. Reagent for the specific determination of pancreatic alpha-amylase in the presence of salivary alpha-amylase in body fluids, especially in serum, plasma, duodenal juice or urine, containing a system for the detection of alpha-amylase and a monoclonal antibody against salivary alpha-amylase, characterised in that it contains a monoclonal antibody which specifically inhibits the salivary enzyme but inhibits the pancreatic enzyme by not more than 50%.

12. Reagent according to claim 11, characterised in that, as system for the detection of pancreatic alpha-amylase, it contains a maltopolyose with 4 to 7 glucose residues, maltose phosphorylase, β-phosphoglucomutase, glucose-6-phosphate dehydrogenase and NAD.

13. Reagent according to claim 11, characterised in that, as system for the detection of pancreatic alpha-amylase, it contains a nitrophenylmaltopolyose with 4 to 7 glucose residues and alpha-glucosidase.

14. Reagent according to claim 11, characterised in that, as system for the detection of pancreatic alpha-amylase, it contains a starch modified with determinable groups.